# EUROPEAN PATENT APPLICATION

(11) **EP 2 789 328 A1**
(43) Date of publication of application: **15.10.2014**
(21) Application number: 13163260.6
(22) Date of filing: 11.04.2013
(51) Int. Cl.: A61J 1/16

(54) **Apparatus for transporting and treating drug containers**

(71) Applicant: Becton Dickinson Holdings Pte. Ltd., Singapore 639461 (SG)
(72) Inventor: Lau, Soo Yong, 06-2839 Singapore 381121 (SG); Jaiswal, Rakesh, 11-355 Singapore 730521 (SG); Lim, Ziv, TECHplace II Singapore 569872 (SG)
(74) Representative: Delorme, Nicolas

(57) **Abstract**

The invention relates to an apparatus (1) for transporting and operating treatments on drug containers (10) comprising :
- an enclosed chamber (11) comprising a storage area (17) and a working area (16) receiving one selected drug container (10a),
- a handling system (20) for moving said selected drug container (10a) from the storage area (17) to the working area (16) and vice versa,
- said working area (16) comprising a first aperture (14) for access to said selected drug container (10a) from the outside by a pull-out system (40) capable of removing the selected drug container (10a) from the enclosed chamber (11), and a second aperture (15) for access to the septum (4) of said selected drug container (10a) by a needle (9a) of a drug administering device (9), and
a cold storage accumulator (13) for maintaining the temperature of said enclosed chamber (11) at a predetermined temperature range.

## Description

The present invention relates to an apparatus for the transportation and handling of drug containers, the containers being used in combination with drug administering devices, such as syringes.

One of the ways to improve health is to immunize entire populations against a number of diseases. To date, injection administration is the most common method of administering vaccines.

Each year, numerous drugs, for example vaccines, need to be prepared throughout the world by healthcare institutions. Many vaccine compositions are usually not stable at room temperature and they must be stored at rather specific cold temperatures. Indeed, due to their biological nature, vaccines are usually temperature sensitive and typically need to be maintained and stored at all time between 2 and 8 degrees Celsius (°C). Some vaccines will be more sensitive to heat exposure and others will be sensitive to freezing. Therefore, maintaining and monitoring the appropriate temperatures during the storage and the handling of vaccines is a critical issue in order to sustain their efficacy. Overexposure to heat as well as overcooling may result in the destruction of the biological elements of the vaccines.

Furthermore, it is critical that the cold chain not be interrupted from the time of production of the drug at a pharmaceutical company to its administration to the patient.

From a supply chain perspective, a vaccine packaging is the multidose container, that is to say, containers that may contain up to 10, 100 or 1000 doses of vaccine, one dose being intended for one patient. Nevertheless, it may happen that a multidose container, such as for example a 10-dose container, is opened and that only three doses are used, for vaccinating three patients, the rest of the container being wasted because it is not administered in a sufficiently short time after opening of the container in order to guaranty the vaccine or drug sterility and its continuous handling at controlled temperatures.

Further complicating appropriate handling of drugs or vaccines, in locations where it is difficult to maintain favorable hygienic conditions such as remote locations which are far from towns and from hospital facilities, the multidose vials may be handled and manipulated at ambiant air, without sterile gloves. Their outer surface may thus become contaminated by outside contaminants, such as bacteria, viruses or funguses that may migrate into the vial. Such a phenomenon may lead to wasting all containers that are not used in a sufficiently short time after opening. Moreover, the outer surface of the vials itself may transmit the outside contaminants to other area by following the path of medical staff, for example during an immunization campaign.

Effective vaccination campaigns can therefore be difficult in some regions and a significant proportion of vaccines may be wasted. This may create an unacceptable cost for the health organizations in charge of immunization campaigns. In addition, it may happen that in case of vaccination campaigns, or pandemic, hundreds of patients need to be vaccinated in a very short time, in locations where it is difficult to maintain good hygienic conditions such as locations which are far from towns and from hospital facilities.

Therefore, it would be desirable to provide an apparatus that would allow for transportation of several medical containers in remote locations while maintaining optimal temperature conditions, and that would allow for maintenance of favorable hygienic conditions during the handling and the filling of several drug administering devices. Such an apparatus would preferably guaranty the continuity of the cold chain which is a critical parameter for the drug compositions contained in multidose containers.

Apparatus for transporting drug containers, provided with refrigeration systems, have been proposed. Nevertheless, they are usually dedicated to store a plurality of containers in the same closed compartment without allowing direct access to a single one container. It would be desirable to provide an apparatus allowing not only transporting a plurality of drug containers, but also operating various tasks on these drug containers, such as inspecting them, for example one at a time, for example for selecting specific vaccine, without compromising the integrity of the other drug containers. In particular, it would be desirable to isolate a drug container from the other stored drug containers so as to complete tasks on said drug container without impeding on the temperature and the sterility of the other drug containers.

In particular, it would be desirable to be able to select and inspect a chosen drug container before withdrawing a dose of product therefrom while avoiding contacts between non-sterile surfaces and the drug containers. In addition, some vaccines need to be mixed before withdrawing a dose of product. It would therefore be desirable to be able to shake the drug container before withdrawing a dose of product, while keeping it sterile as much as possible at the desired temperature.

There is therefore room for improvement in the field of storing, transporting and administering vaccines in regions where electricity supply is limited or nonexistent.

It is an object of the present invention to provide an apparatus for storing, transporting and operating some tasks or treatments on drug solutions such as vaccines with relatively little or no electricity supply.

It is a further object of the present invention to provide an apparatus for storing and transporting drug solutions such as vaccines with relatively little or no electricity supply while maintaining the drug sanitary state during and after the withdrawal of at least one dose.

A first aspect of the present invention is an apparatus for transporting and operating treatments on a plurality of drug containers filled with a drug solution and closed by a septum, the apparatus comprising :
- an enclosed chamber comprising a storage area for storing said plurality of drug containers and a working area capable of receiving one drug container selected from said plurality of drug containers,
- a handling system for moving said selected drug container from the storage area to the working area and vice versa,
- said working area comprising a first aperture to allow access to said selected drug container from the outside of said enclosed chamber by a pull-out system capable of removing the selected drug container from the enclosed chamber, and a second aperture to allow access to the septum of said selected drug container by a needle of a drug administering device, and
- a cold storage accumulator for maintaining the temperature of at least said enclosed chamber at a predetermined temperature range.

The apparatus in accordance with an embodiment of the invention allows for storing, transporting and operating treatments on drug containers, such as for example removing a selected drug container from the apparatus for inspecting it or shaking it, or withdrawing a dose of product from said drug container while ensuring favorable hygienic and temperature conditions. These operations can be realized without having to remove other drug containers also contained in the apparatus, while maintaining all the drug containers at a desired temperature range ensuring continuous efficiency and integrity of the drug solution contained in the drug containers. Moreover, this apparatus allows for the realization of the various different steps required before an injection of a drug without having to handle directly the selected drug container with hands, said drug container remaining inside the apparatus. In addition, the apparatus in accordance with an aspect of the invention allows completing these operations without electrical supply, in regions where no electrical power may be found. In addition, this apparatus with its working area dedicated to one drug container ensures that the user selects the correct drug and removes it from the container without any misuse.

In embodiments, the apparatus further comprises a locking system for temporary locking operation of the handling system when one selected drug container is positioned in said working area. In such embodiments, the user may complete the tasks and/or treatment he is intended to perform without having to fear that the drug container may move from its position. The expected drug is therefore withdrawn from its container in safe condition. At the same time, due to the apparatus of the invention, the user knows the other drug containers contained in the apparatus are maintained at the desired temperature.

In embodiments, the apparatus further comprises an enclosed compartment capable of receiving said pull-out system before any selected drug container is positioned in said working area, said compartment being located adjacent said enclosed chamber and being provided with a first access port in communication with said first aperture and with a second access port in communication with the outside, said first and second access ports being configured so as to allow removal of said pull-out system and selected drug container from said apparatus. The user needs not looking for a pull-out system for removing the drug container he wishes to work on, for example for inspection, shaking and/or withdrawing a dose of product. Such an integrated pull-out system simplifies the operations performed by the user and significantly limits the handling of the drug containers when inspection, shaking and/or withdrawing are required before withdrawal of the drug from the container. All these different steps can be done without direct contact with the selected drug container.

In embodiments, at least part of said cold storage accumulator is located within said enclosed compartment. The pull-out system is therefore itself maintained at the desired temperature before use. Risks that the temperature of the enclosed chamber gets out of the desired or necessary range (for vaccines in particular) are therefore greatly limited.

In embodiments, the apparatus further comprises a cap for releasingly closing said second access port from the outside. The enclosed chamber, as well as the enclosed compartment, is therefore thermally sealed.

In embodiments, the apparatus further comprises a first surface located on or coupled to said selected drug container, and a second surface located on said pull-out system, said first and second surfaces being capable of cooperating for automatically engaging each other when said selected drug container is moved from the storage area to the working area and for automatically disengaging from each other when said selected drug container is moved from the working area to the storage area. For example, the first surface defining a projecting head, the second surface may define a transversal slit capable of receiving said projecting head with engagement. In particular, the first and second surfaces engage each other so as to couple the pull-out system to the selected drug container in the direction of the removal of the drug container by the pull-out system. The user therefore does not need to fix the pull-out system to the selected drug container and he does not need to detach the pull-out system from the selected drug container when the operations he had to perform on said selected drug container are over, as these steps are completed automatically when he selects the drug container and causes it to move towards or away from the working area. This automatic coupling between the selected drug container and the pull-out system allows a simple, safe and clean displacement of the drug container within the apparatus as it limits the direct handling of the drug container by the user. Indeed, once the first surface and the second surface are engaged together, the user simply needs to pull on the pull-out system to withdraw the selected drug container from the enclosed chamber.

In embodiments, said first surface being a metal surface, said second surface is a magnet capable of magnetizing said first surface. Alternatively, the first and second surfaces may be two complementary surfaces cooperating together via a mechanical engagement.

In embodiments, each drug container being fixedly received in a holder, said first surface is located on said holder. The holder may further serve to protect the drug container from shocks, and/or from light and/or from heat.

In embodiments, said second surface being provided on a removal member of said pull-out system, said pull-out system further comprises a protecting sleeve coupled to said removal member, said protecting sleeve being capable of moving with respect to said removal member at least when said pull-out system and said selected drug container are engaged together, so as to extend around said selected drug container when said pull-out system removes said selected drug container from said working area of said enclosed chamber.

The drug container is therefore double protected for example from shocks, light and/or heat when it is removed from the enclosed chamber, for example for inspection by the user, in particular due to said holder and protecting sleeve. In embodiments, the protecting sleeve is formed of an insulated material. The temperature of the drug container, while it is out of the enclosed chamber, is therefore maintained to the desired or necessary range for ensuring the integrity of the drug solution contained therein.

In embodiments, the holder may be formed of an insulated material. For example, the holder and/or the protecting sleeve may be configured so as to allow the user to see the inside of the drug container. For example, the holder and/or the protecting sleeve may be made of a transparent material. Alternatively or in combination, the holder and/or the protecting sleeve may be provided in their side walls with one or more windows for viewing the drug container contents.

In embodiments, the apparatus further comprises a shaking mechanism allowing the user to shake the selected drug container within said working area of said enclosed chamber when said selected drug container is engaged with said pull-out system. The user may then proceed to the mixing of the contents of the drug container without fearing that the outside temperature adversely affects the drug container contents and jeopardizes the integrity of the vaccine and/or drug solution, and without having to take the drug container in his hands.

In embodiments, said selected drug container being received in abutment against a bottom wall of a lodging of said handling system when said selected drug container is positioned in said working area, said shaking mechanism comprises an elastic component provided between said selected drug container and said bottom wall. The elastic component acts as elastic return means, so that, when the user pushes on the selected drug container in the direction of the bottom wall, for example via the pull-out system, the elastic component is compressed. When the user releases his pressure on the selected drug container, the elastic component comes back rapidly to its initial rest state. As a consequence, a succession of pushes and releases by the user causes back and forth movements of the selected drug container. The selected drug container is shaken and its content is mixed. The shaking is enhanced and more efficient due to the presence of the elastic component. For example, the elastic component is a helical spring positioned between the bottom wall of the lodging and the drug container.

In embodiments, the apparatus further comprises a docking compartment configured for receiving said drug administering device and provided with an opening in communication with said second aperture for allowing the needle of said drug administering device to enter the septum of the selected drug container positioned in said working area.

In embodiments, the apparatus is further provided with one or more temporary closure(s) for closing one or several of said first and second apertures and of said first and second access ports and said opening, when no treatment is operated on the drug containers, so as to maintain said enclosed chamber and enclosed compartment thermally sealed. Such closure(s) allow maintaining the enclosed chamber and the enclosed compartment free from any outside contaminants by avoiding any contamination from outside air or dust.

The following detailed description of several embodiments of the invention is better described when read in conjunction with the appended drawings being understood that the invention is not limited to the specific embodiments disclosed.
Figure 1 is a perspective view of an apparatus in one embodiment of the present invention;
Figure 2 is a top view of the apparatus of Figure 1 after the top lid has been removed;
Figure 3 is a partial perspective and section view of the apparatus of Figure 2;
Figure 4 is a perspective view of the handling system of the apparatus of Figures 2 and 3;
Figure 5 is an exploded view of the handling system of Figure 4;
Figure 6 is a bottom view of the handling system of Figure 4;
Figures 7A-C are partial bottom views of the locking system of the handling system of Figures 4-6;
Figure 8 is a partial section view of the apparatus of Figure 3 with the pull-out system located within the enclosed compartment;
Figures 9A and 9B are respectively a partial perspective view and a partial exploded view of the handling system and the pull-out system of the apparatus of Figure 3;
Figure 10 is a side view of the removal member of the pull-out system of Figures 9A and 9B;
Figure 11 is a partial side view of the removal member of Figure 10 engaged with a holder of a drug container;
Figure 12 is a partial side view of the removal member and holder of Figure 11 together with a shaking system;
Figures 13 and 14 are side views illustrating the inspection step, respectively when the drug container is removed from the enclosed chamber, and when the drug container is removed from the apparatus;
Figure 15 is a perspective view of a drug administering device to be filled with a dose of the drug of the drug containers of the apparatus of Figure 3;
Figure 16 is a perspective view of the apparatus of Figure 1 with side door open showing the docking compartment.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures.

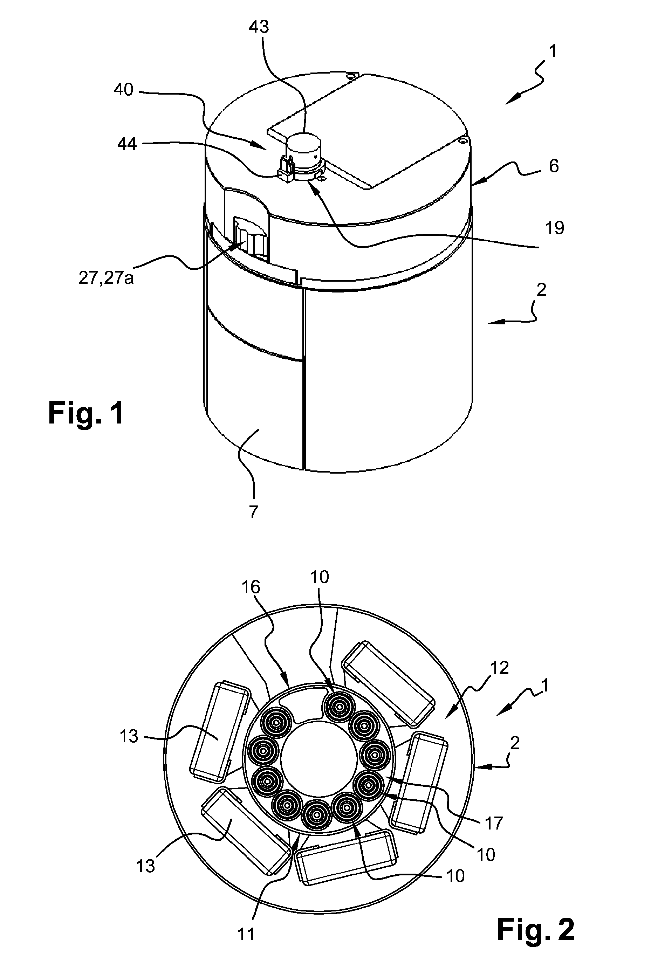

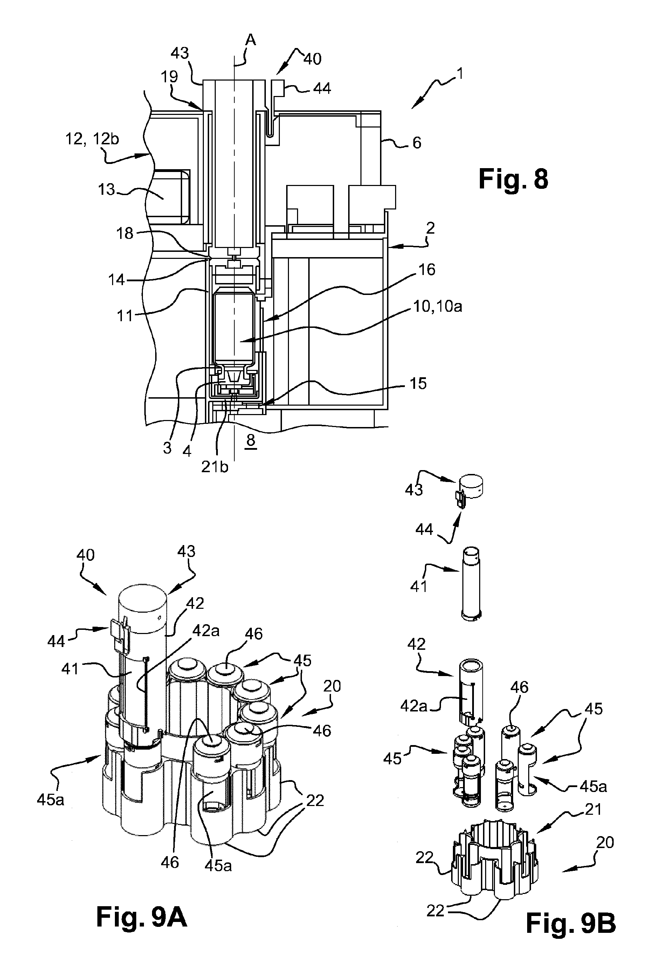

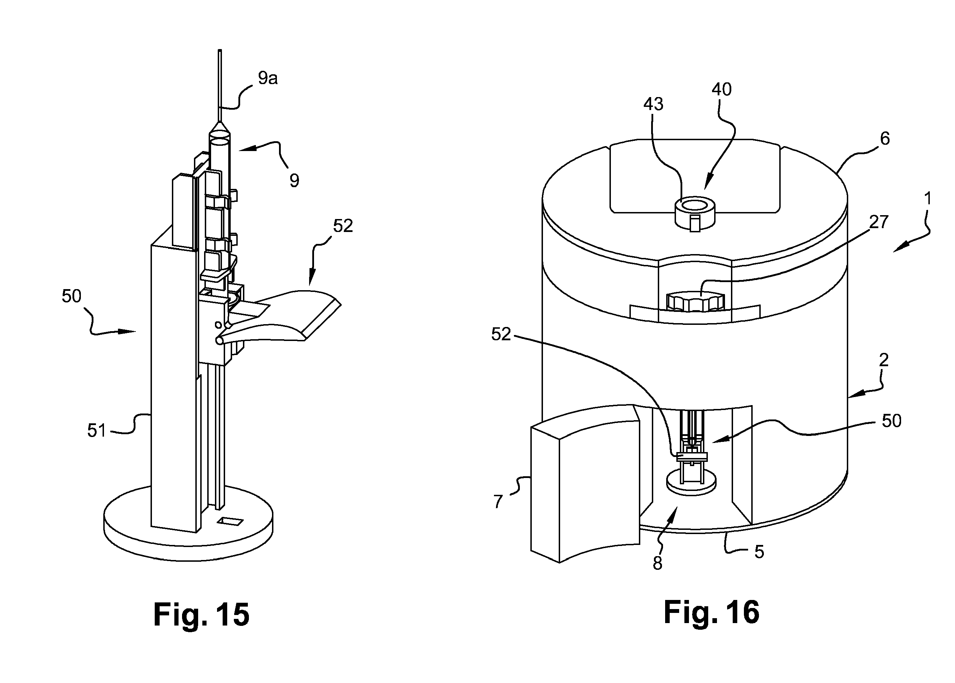

With reference to Figures 1-3, 8 and 16, an apparatus 1 is shown in accordance with an embodiment of the invention for storing, transporting and operating treatments or tasks on a plurality of drug containers, such as multidose vials 10, filled with a drug solution such as vaccine, while maintaining the appropriate temperature and hygienic conditions for preserving the integrity and efficiency of the drug solution. With reference to Figure 8, a vial 10 comprises a tubular barrel having a longitudinal axis, closed at an end and having a collar 3 at the opposite end, said collar 3 forming an opening closed by a septum 4. The septum 4 is usually made of a material impermeable to gas and liquid and it seals hermetically the content of the vial 10. The septum 4 has an outer surface which is exposed to the outside and which can be pierced by the needle of a drug administering device to withdraw a dose of drug from the vial 10, as will be shown later with reference to Figures 15 and 16.

In the illustrated embodiment of the invention, the apparatus 1 comprises a substantially cylindrical housing 2 closed at its bottom with a transversal wall 5 (see Figure 16) and at its top by a top lid 6. However, alternative external geometries can be appropriate depending on storage and transportation requirements.

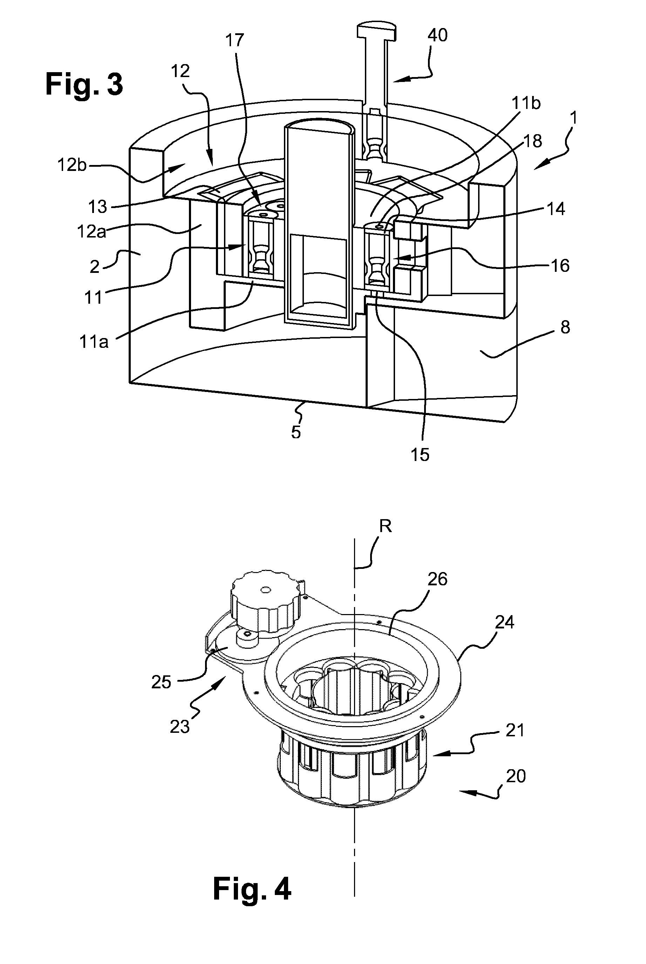

With reference to Figure 2, which shows the apparatus 1 with the top lid 6 removed for clarity, the apparatus 1 further comprises an enclosed chamber 11 located in the center of the cylindrical housing 2, this enclosed chamber 11 receiving a plurality, ten on the example shown, of vials 10. With further reference to Figure 3, the apparatus 1 further comprises an enclosed compartment 12 located adjacent to the enclosed chamber 11, and comprising a cylindrical part 12a and a top part 12b.

The cylindrical part 12a of the enclosed compartment 12 surrounds the enclosed chamber 11 and receives a plurality, five on the example shown in Figure 2, of cold storage accumulators 13. The cold storage accumulators 13 can contain an eutectic fluid which can be frozen in a refrigerating equipment such as a refrigerator and then releases cooling energy to its surrounding environment. The cold storage accumulators 13 are intended to maintain at least the enclosed chamber 11 at a predetermined temperature range. In the case of vaccines, this temperature range should be between 2°C and 8°C for preserving the integrity and efficiency of the vaccines contained in the vials 10.

The top part 12b of the enclosed compartment 12, located above the enclosed chamber 11, receives a pull-out system 40 for removing vials 10 from the enclosed chamber 11 and from the apparatus 1. The pull-out system 40 is actionable by a user and will be described in detail later on. The pull-out system 40 also serves for repositioning a vial 10 inside the enclosed chamber 11, once inspection and/or other necessary treatments have been performed on the vial 10 previously removed. The top part 12b of the enclosed compartment 12 may also receive additional cold storage accumulators 13, as shown in Figure 8.

With reference to Figure 16, the apparatus 1 further comprises a docking compartment 8, located underneath the enclosed chamber 11, where a drug administering device such as a syringe 9 (see Figure 15) can be secured and can be connected to a selected vial 10 of the enclosed chamber 11 to withdraw a dose of drug solution, as will be described below. The cylindrical housing 2 is therefore provided with a side door 7 opening onto the docking compartment 8.

The walls of the housing 2 can be suitably made of an insulating material such as, for example, a sandwich of different layers having a hard and smooth surface on the outside for durability and hygiene and internal layer(s) of for example polystyrene foam for insulation purpose. In the illustrated embodiment, the top lid 6 is snap-fitted onto the cylindrical housing 2, and the side door 7 is hinged onto the cylindrical housing 2. Other securing arrangement of the top lid 6 and side door 7 onto the housing 2 can be contemplated. The side door 7 is also suitably equipped with locking devices to make sure that it cannot be unintentionally opened.

With reference to Figure 3, the enclosed chamber 11 will now be described in details. The enclosed chamber 11 has globally the shape of a cylindrical cavity provided with a bottom wall 11a and a top wall 11b. It is thermally sealed except for two apertures, a first aperture 14 provided in the top wall 11b, and a second aperture 15 provided in the bottom wall 11a and facing the first aperture 14. The part of the enclosed chamber 11 located between the first aperture 14 and the second aperture 15 defines a working area 16 of the enclosed chamber 11, whereas the rest of the enclosed chamber 11 defines a storage area 17 of the enclosed chamber 11.

As will be clear from the description below, the storage area 17 is intended to receive the plurality of vials 10 for storage and transportation, whereas the working area 16 is intended to receive one vial 10 at a time, hereinafter referred to as the selected vial 10a, for submission to various treatments such as inspection, shaking, withdrawal of a dose of drug solution contained therein. In this view, the first aperture 14 is dimensioned and shaped so as to allow access to, and removal of, the selected vial 10a located in said working area 16, by the pull-out system 40. The second aperture 15 is dimensioned and shaped so as to allow access to the septum 4 of the selected vial 10a by the needle 9a of a drug administering device 9, as shown in Figures 15 and 16. In case the docking compartment 8 comprises a top wall adjacent the bottom wall 11a of the enclosed chamber 11, the top wall of the docking compartment may be provided with an opening in communication with the second aperture 15 for allowing the needle of the drug administering device 9 to enter the septum 4 of the selected vial 10a. In embodiments not shown, a connector may be provided on the collar 3 of each vial 10 in order to limit the contamination of the septum 4 as several drug administering device needles 9a will pierce successively the septum 4.

Closure means, such as doors or hatches, may be provided for temporary closing the first and second apertures (14, 15) in view of optimizing the refrigeration and the hygienic conditions of the enclosed chamber 11 when no treatment is performed on the selected vial 10a or when no selected vial is present in the working area 16 of the enclosed chamber 11. For example, closure means of the second aperture 15 may be opened for drug solution withdrawal while closure means of the first aperture 14 are closed to maintain the cooling capacity of the cold storage accumulators 13. On the contrary, when the inspection and/or shaking steps are performed on the selected vial 10a, the closure means of the second aperture may be closed to maintain the cooling capacity of the cold storage accumulators 13 and to avoid contamination from outside air or dust. The closure means are preferably made of an insulating material so as to maintain the vials 10 in an insulated environment.

The apparatus 1 has thus an enclosed chamber 11 where vials 10 can be stored in favorable hygienic conditions under a passively maintained temperature.

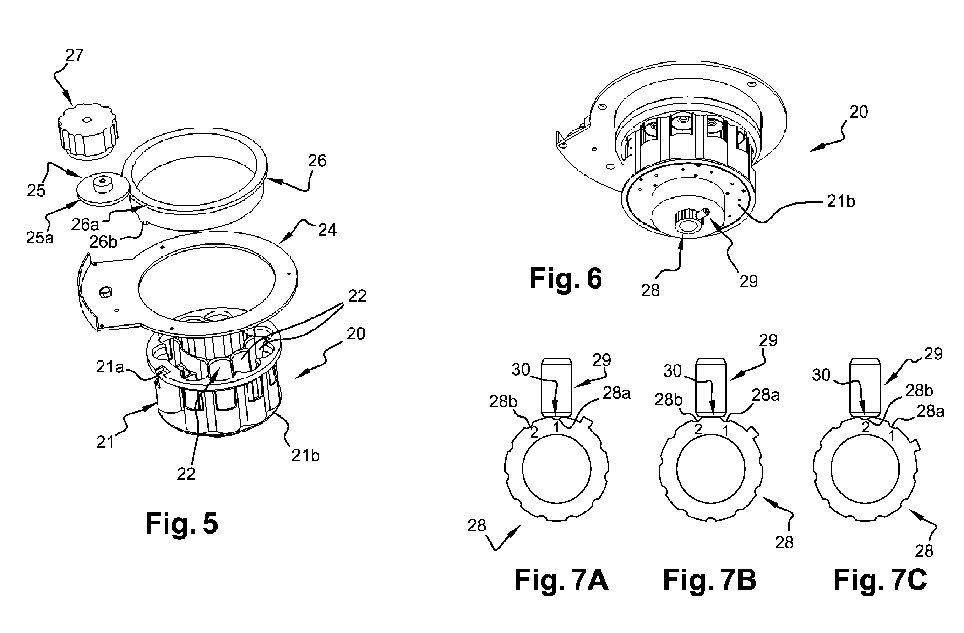

The enclosed chamber 11 further comprises a handling system for moving the selected vial 10a from the storage area 16 to the working area 17 and vice versa. In the illustrated embodiment, the handling system comprises a carrousel 20 capable of rotating around its rotational axis R by means of a gear 23 actionable by a user. With reference to Figures 4 and 5, the carrousel 20 comprises a cylindrical basket 21 closed at its bottom by a transversal wall 21b and comprising a plurality of circumferentially distributed lodgings having the shape of nests 22, each nest 22 being capable of receiving a vial 10, with the septum 4 and collar 3 of the vial 10 facing the transversal wall 21b. The gear 23 comprises a ring 24, intended to be fixed with respect to the apparatus 1, this ring 24 receiving a first wheel 25 provided with outer teeth 25a capable of cooperating with the outer teeth 26a of a second wheel 26. The second wheel 26 is coupled to the cylindrical basket 21 via a peg 26b engaging a recess 21a of the cylindrical basket 21. The first wheel 25 is further coupled to a button 27 capable of being rotated, a part 27a of which protrudes outside the housing 2 (see Figure 1) and is accessible by the user.

When the user rotates the button 27, the first wheel 25 rotates and thereby causes the rotation of the second wheel 26 and of the carrousel 20. Rotation of the carrousel 20 causes circular movement of the cylindrical basket 21 and therefore of all the nests 22. One of the nests, receiving the selected vial 10a, is brought to the working area 16 of the enclosed chamber 11. Once the selected vial 10a is in the working area 16, it is aligned on a longitudinal axis A joining first aperture 14 to second aperture 15, this longitudinal axis being also the longitudinal axis of the pull-out system as will be shown later (see Figure 8). In this position, the outer surface of the septum 4 of the selected vial 10 faces the second aperture 15 of the working area 16.

In the illustrated embodiment, the apparatus 1 further comprises a locking system for temporary locking operation of the handling system, in particular for temporary locking activation of the button 27 by the user, in order to maintain the nest 22 receiving the selected vial 10a in its position in the working area 16 for the time required by the user for performing the tasks he wishes to perform on the selected vial 10a.

With reference to Figures 6 and 7A-C, the locking system comprises a cogwheel 28 located at the center of the outer face of the transversal wall 21b of the cylindrical basket 21 and a sleeve 29, extending radially from the cogwheel 28 and intended to be fixed with respect to the apparatus 1 (not shown). The sleeve 29 encloses a peg 30 coupled to elastic return means (not shown) capable of moving the peg 30 inside and out of said sleeve 29 under the effect of a stress exerted on the peg 30.

With reference to Figures 5, 7A and 8, when a nest 22, and the corresponding selected vial 10a, is in position in the working area 16 of the enclosed chamber 11, the peg 30 is engaged in a first recess 28a (position named 1 in Figure 7A) of the cogwheel 28 and the elastic return means are for example in abutment against the peg 30 and in a rest state. Therefore, moving the peg 30 out of recess 28a requires a specific effort from the user on the button 27. As long as the user does not exert this specific effort, the nest 22 positioned in the working area 16 will not move, as the basket 21 is blocked in rotation by means of the peg 30 being engaged in the recess 28a. When the user decides that no more treatment is to be performed on the selected vial 10a, he rotates button 27 by applying the necessary effort for disengaging the peg 30 from the recess 28a. During this step, as shown in Figure 7B, the peg 30 retracts within the sleeve 29 and causes the elastic return means to be compressed. The basket 21 rotates until the peg 30 faces the next recess 28b and is pushed in engagement therewith (position named 2 in Figure 7C) by means of the elastic return means coming back to their rest state, as shown in Figure 7C. Another nest 22, adjacent the first nest 22, is now in position in the working area 16 of the enclosed chamber 11, and the user may complete new tasks on a new selected vial 10a present in this new nest 22.

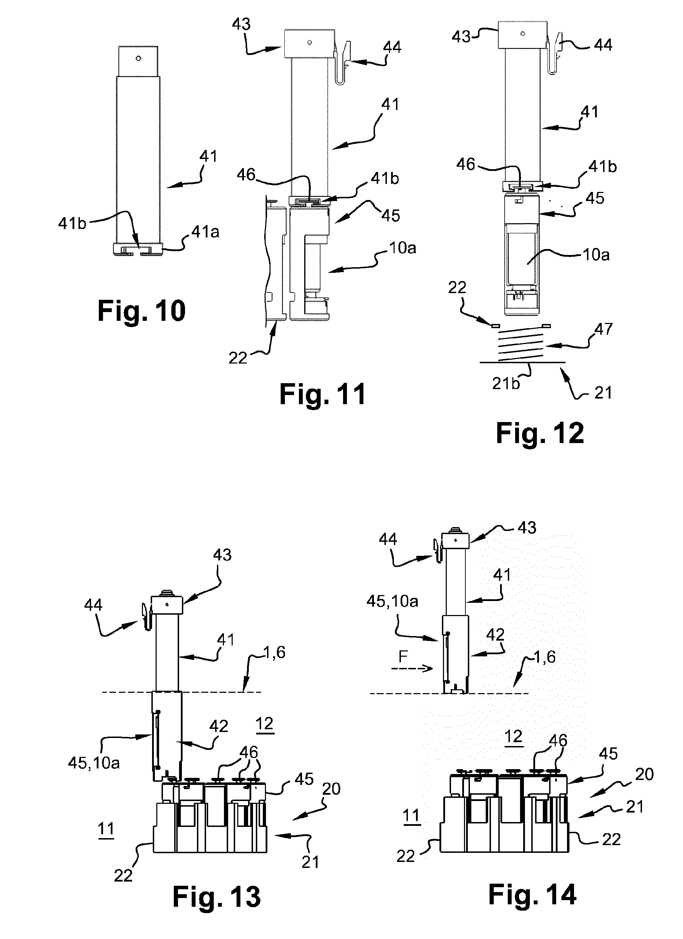

With reference to Figures 9A to 11, the pull-out system 40 will now be described in detail. The pull-out system 40 comprises a removal member under the form of an elongated tube 41 intended to engage the selected vial 10a at time of removal or repositioning of the selected vial 10a from or inside the enclosed chamber 11. As shown in Figure 10, he bottom end of the elongated tube 41 is provided with an outer rim 41a in which is designed a surface defining a transversal slit 41b, the function of which will be explained later on. The pull-out system 40 further comprises a sleeve 42 surrounding the elongated tube 41, and provided with a longitudinal window 42a for allowing a user to see inside the sleeve 42. The top end of the sleeve 42 comprises an inner rim (not visible in the figures). The sleeve 42 is movable with respect to the elongated tube 41 between a retracted position in which it significantly surrounds the elongated tube 41, as shown in Figure 9A, and an extended position (see Figures 13 and 14), in which the inner rim of its top end is in abutment on the outer rim 41a of the elongated tube 41. The pull-out system 40 further comprises a cap 43 covering and snap-fitted to the top part of the elongated tube 41 : in the position where the pull-out system 40 is not in use and the selected vial 10a is not removed from the enclosed chamber 11, as shown in Figures 8 and 9A, the cap 43 is coupled to the sleeve 42 by means of a latch 44 so that the sleeve 42 is in its retracted position and surrounds the elongated tube 41. As shown later on, the latch 44 is releasable by a user to allow the sleeve 42 to reach its extended position.

With reference to Figures 9A-11, each vial 10 is fixedly received in a holder 45. The holder 45 allows a simplified handling of the vial 10 and provides protection to the vial, for example against shocks and contamination, during said handling. The holder 45 has the shape of a sleeve closed at its both ends and receiving the vial 10. The holder 45 may be made of an insulated material for protecting the vial 10 from heat. In particular, the vial 10 is blocked in longitudinal translation with respect to the holder 45 once it is received therein. The holder 45 is provided with a side window 45a for allowing the user to see inside the holder, in particular to see the vial 10 and its contents. The top end of each holder 45 is provided with a surface defining a projecting head 46.

The use of the apparatus 1 will now be explained in detail.

Vials 10 received in their holders 45 are inserted into the nests 22 of the carrousel 20 of the enclosed chamber 11. In each nest 22, the holder 45 is in abutment against the transversal wall 21b of the cylindrical basket 21 of the carrousel 20. The cold storage accumulators 13 which have been previously stored in a refrigeration system, so that the eutectic fluid is preferably entirely solidified, are inserted into the enclosed compartment 12. The vials 10 are thus surrounded by a cooling capacity.

As shown in Figure 8, the pull-out system 40 is stored in the top part 12b of the enclosed compartment 12. The enclosed compartment 12 has a first access port 18 in communication with the first aperture 14 and a second access port 19 in communication with the outside. The bottom end of the pull-out system 40 faces the first access port 18 and its top end, namely the cap 43, protrudes outside the apparatus 1 via the second access port 19. Before any use of the apparatus 1, the cap 43 closes the second access port 19 for maintaining the enclosed compartment 12 thermally sealed. For the same reason, the first aperture 14 and the first access port 18 may be closed for maintaining the enclosed chamber 11 thermally sealed and uncontaminated.

The top lid 6 is closed and preferably locked to avoid any contact with ambient air, at a potentially much higher temperature and likely to carry some contaminants. The temperature within the enclosed chamber 11 is thus remained between 2 degrees Celsius and 8 degrees Celsius over a period of time, for example 10 to 12 days, and the enclosed chamber remains in favorable hygienic conditions.

In this way, the apparatus 1 can be carried to any places where there is relatively little or limited electricity supply without damaging the content of the vials 10 as the enclosed chamber 11 is kept at the correct temperature.

Furthermore, the apparatus 1 allows the delivery of drugs and vaccines to people living in remote area, even if dust and contaminants are present, such as in deserts, equatorial or tropical areas, and where no medical infrastructure is available.

When a user decides to perform an operation on a vial 10 stored in the apparatus 1, he first causes opening of the first aperture 14 and of the first access port 18, so that the first aperture 14 and the first access port 18 are in communication with each other. In particular, the transversal slit 41b of the elongated tube 41 of the pull-out system 40 now protrudes inside the working area 16 of the enclosed chamber 11.

The user then decides to select a vial 10 for performing one of the following operations : inspection of the vial 10, and/or shaking the vial 10, and/or proceeding to the withdrawal of a dose of drug solution from said vial 10. The user rotates the button 27 in order to bring a selected vial 10a in the working area 16 of the enclosed chamber 11. Due to the locking system formed by the peg 30 and the cogwheel 28, the selected vial 10a is caused to be maintained in the right position in the working area 16, aligned onto the longitudinal axis A of the pull-out system 40, as shown in Figure 8. During circular movement of the nest 22 receiving both the selected vial 10a and its holder 45, the projecting head 46 of the holder 45 has cooperated with, and has engaged, the transversal slit 41b of the elongated tube 41, as shown in Figure 11. In Figure 11, the sleeve 42 of the pull-out system 40 is not represented for sake of clarity. As a consequence of the projecting head 46 being engaged in the transversal slit 41b, the removal member (elongated tube 41) of the pull-out system 40 is now engaged with the selected vial 10a, as shown in Figure 8. The engagement of the pull-out system 40 with the selected vial 10a therefore occurs automatically as the user rotates the button 27. The user needs not to perform additional step than rotating the button 27. No opening of the enclosed chamber 11 is required, as well as no direct contact between the user and the selected vial 10a. All the stored vials 10a are thereby within a desirable temperature range and free of outside contamination.

For being able to remove the selected vial 10a from the enclosed chamber 11 in a view of inspecting its contents, the user then releases the latch 44 of the pull-out system 40, and he pulls up the elongated tube 41 by means of the cap 43 which is coupled to the elongated tube 41, as shown in Figure 13. The latch 44 being released, the sleeve 42 is no more maintained in its retracted position with respect to the elongated tube 41. As a consequence, as the user pulls up on the cap 43, the holder 45 and the vial 10a are drawn in the top direction with the elongated tube 41. In a first step, the sleeve 42 remains in the enclosed compartment 12 and extends progressively around the holder 45 and the vial 10a, as shown in Figure 13, on which the top wall of the lid 6 of the apparatus 1 is schematically represented by a dotline. The sleeve 42 thereby forms an additional protection for the selected vial 10a, as it is removed from the enclosed chamber 11.

In order to be able to inspect the selected vial 10a, the user continues pulling on the cap 43 and the inner rim of the sleeve 42 comes in abutment against the outer rim 41a of the elongated tube. By continuing his movement, the user eventually removes the holder 45 and the vial 10a from the apparatus 1 and is allowed to see the vial 10a and its contents via the windows (45a, 42a) of both the holder 45 and the sleeve 42, as shown by the arrow F in Figure 14, on which the top wall of the lid 6 of the apparatus 1 is schematically represented by a dotline. Although the vial 10a is now outside the apparatus, it is protected both by the holder 45 and the sleeve 42, which may be both formed of insulated material. As such, the user has the time to inspect the contents of the vial 10a, without fearing that the temperature of the vial 10a rises above the desired range of 2-8°C for a vaccine for example. Moreover, with such holder and sleeve, the user does not need to touch directly the selected vial 10a, preserving it from contamination by direct contact with unclean hands or non-sterile gloves The user may rotate the selected vial 10a for a better inspection if necessary.

In another embodiment not shown, the transversal slit of the pull-out system 40 may be replaced by a metal surface. In such a case, the projecting head 46a of the holder 45 may comprise a magnet, able to cooperate with the metal surface. In such an embodiment, the engagement between the elongated tube 41 and the holder 45 is thus achieved as a result of magnetic forces, the operation of the pull-out system remaining unchanged.

With reference to Figure 12, is shown an embodiment where a helical spring 47 is located in the nest 22 between the bottom end of the holder 45 of the vial 10a and the bottom transversal wall 21b of the cylindrical basket 21. When the pull-out system 40 is engaged with the holder 45 by means of projecting head 46 being engaged in transversal slit 41b, and the latch 44 is released, the user may apply a pressure on the pull-out system 40 in the direction of the bottom wall 21b of the cylindrical basket 21. Under such a pressure, the helical spring 47 is compressed. When the user releases his pressure, the helical spring 47 tends to come back naturally to its rest state. A succession of such pressures and releases by the user therefore causes the shaking of the selected vial 10a and the mixing of its contents. During this shaking, the vial 10a is globally maintained inside the enclosed chamber 11 and there is therefore no risk that its temperature rises above the desired temperature range, for example 2-8°C for a vaccine. Moreover, the shaking is being made inside the enclosed chamber 11, exposition of the selected vial 10 to outside contaminants is avoided.

Once a selected vial 10a has been inspected, shaken if necessary, and repositioned it in the working area 16 by means of the pull-out system 40, the user may proceed to the step of withdrawing a dose of drug solution from the selected vial 10a, with the help of a drug administering device 9 as shown in Figures 15 and 16.

With reference to Figure 15 is shown a drug administering device 9, such as a syringe, provided with a needle 9a. The drug administering device 9 may be secured on a vertical shaft 51 of a loading system 50 intended to be positioned in the docking compartment 8 so that the needle 9a faces the outer surface of the septum 4 of the selected vial 10a (see Figures 8 and 16). The user then operates an actuator 52 of the loading system 50 in order to push the needle 9a towards the septum 4 so that the needle 9a pierces the septum 4 and enters inside the selected vial 10a. The user then causes the stopper (not visible in the figures) of the drug administering device 9 to move backwards by pushing backwards the actuator 52, in order to fill said device with a dose of drug solution from the selected vial 10a. He then removes the drug administering device 9 from the loading system 50 and proceeds to the injection of the dose of the drug solution to the patient.

Once all the doses of vaccine or drug solution contained in a first selected vial 10a have been withdrawn, or if the user wishes to fill a drug administering device with a drug solution from another vial 10 located in the storage area 17, the user further rotates the button 27. The circular movement of the cylindrical basket 21 thus causes the disengagement of the projecting head 46 of the holder 45 from the transversal slit 41b of the elongated tube 41. While the circular movement of the cylindrical basket 21 continues, the projection head 46 of the holder 45 of the adjacent nest 22 becomes automatically engaged in the transversal slit 41b of the elongated tube 41 of the pull-out system 40. The user may then repeat the tasks described above on the new selected vial 10a.

Additionally, the apparatus 1 can be equipped with an electronic terminal which can store data such as the type of drug, the date of administration, the number of doses, the type of population etc. For example, a mechanical vial indicator may be used to indicate to the user which vial 10 is present in the working area 16. This could be realized by coupling the second wheel 26 of the handling system with a numbered disk capable of indicating which vial number is selected. In such a case, the number could be seen by the user by a specific window (not shown) present for example in the cylindrical part 12a or in the top part 12b of the enclosed compartment 12.

The described apparatus thus provides a significant improvement as it is possible to transport temperature sensitive drugs such as vaccines stored in multidose containers in an autonomous way, and to complete various tasks and/or operations on a selected container without fearing that the temperature of the selected container rises dramatically above an undesired temperature limit during these treatment steps. Furthermore, the invention allows for preservation of favorable hygienic conditions around the drug containers as manual handling of the drug containers outside of the apparatus is not required. In other terms, the apparatus according to the invention is specifically adapted for transporting and treating temperature sensitive drugs in area where access to electricity is limited or nonexistent and where hygienic conditions required for immunization or cure of patients cannot be sustained.

In addition, wastage of unused doses stored in an opened multidose vial is avoided, as the apparatus described allows for isolating the vial from outside dust, air contaminants and contaminated surfaces. This maintains efficacy of the contained drug or vaccine overtime and allows using the global content of a multidose vial overtime.

In addition, by limiting the contaminants reaching directly the surface of the containers, the apparatus of the invention allows limiting or preventing the propagation of diseases from an immunization site to another.

## Claims

1. An apparatus (1) for transporting and operating treatments on a plurality of drug containers (10) filled with a drug solution and closed by a septum (4), the apparatus comprising :
- an enclosed chamber (11) comprising a storage area (17) for storing said plurality of drug containers and a working area (16) capable of receiving one drug container selected from said plurality of drug containers,
- a handling system (20) for moving said selected drug container from the storage area to the working area and vice versa,
- said working area comprising a first aperture (14) to allow access to said selected drug container from the outside of said enclosed chamber by a pull-out system (40) capable of removing the selected drug container from the enclosed chamber, and a second aperture (15) to allow access to the septum of said selected drug container by a needle (9a) of a drug administering device (9), and
- a cold storage accumulator (13) for maintaining the temperature of at least said enclosed chamber at a predetermined temperature range.

2. The apparatus (1) of claim 1, further comprising a locking system (28, 29, 30) for temporary locking operation of the handling system when one selected drug container is positioned in said working area.

3. The apparatus (1) of claim 1 or 2, further comprising an enclosed compartment (12) capable of receiving said pull-out system before any selected drug container is positioned in said working area, said compartment being located adjacent said enclosed chamber and being provided with a first access port (18) in communication with said first aperture and with a second access port (19) in communication with the outside, said first and second access ports being configured so as to allow removal of said pull-out system and selected drug container from said apparatus.

4. The apparatus (1) of claim 3, wherein at least part of said cold storage accumulator is located within said enclosed compartment.

5. Apparatus (1) of claim 3 or 4, further comprising a cap (43) for releasingly closing said second access port from the outside.

6. Apparatus (1) of any one of claims 3 to 5, further comprising a first surface (46) located on or coupled to said selected drug container, and a second surface (41b) located on said pull-out system, said first and second surfaces being capable of cooperating for automatically engaging each other when said selected drug container is moved from the storage area to the working area and for automatically disengaging from each other when said selected drug container is moved from the working area to the storage area.

7. Apparatus (1) of claim 6, wherein said first surface defining a projecting head (46), said second surface defines a transversal slit (41b) capable of receiving said projecting head with engagement.

8. Apparatus (1) of claim 6 or 7, wherein said first surface being a metal surface, said second surface is a magnet capable of magnetizing said first surface.

9. Apparatus (1) of any one of claims 6 to 8, wherein each drug container being fixedly received in a holder (45), said first surface (46) is located on said holder.

10. Apparatus (1) of any one of claims 6 to 9, wherein said second surface (41b) being provided on a removal member (41) of said pull-out system, said pull-out system further comprises a protecting sleeve (42) coupled to said removal member, said protecting sleeve being capable of moving with respect to said removal member at least when said pull-out system and said selected drug container are engaged together, so as to extend around said selected drug container when said pull-out system removes said selected drug container from said working area of said enclosed chamber.

11. Apparatus (1) of claim 10, wherein the protecting sleeve is formed of an insulated material.

12. Apparatus (1) of any one of claims 1 to 11, further comprising a shaking mechanism (47) allowing the user to shake the selected drug container within said working area of said enclosed chamber when said selected drug container is engaged with said pull-out system.

13. Apparatus (1) of claim 12 wherein said selected drug container being received in abutment against a bottom wall (21b) of a lodging (22) of said handling system when said selected drug container is positioned in said working area, said shaking mechanism comprises an elastic component (47) provided between said selected drug container and said bottom wall.

14. Apparatus (1) of any one of claims 1 to 13, further comprising a docking compartment (8) configured for receiving said drug administering device (9) and provided with an opening in communication with said second aperture for allowing the needle (9a) of said drug administering device to enter the septum of the selected drug container positioned in said working area.

15. Apparatus (1) of any one of claims 1 to 14, further provided with one or more temporary closure(s) for closing one or several of said first and second apertures and of said first and second access ports and said opening, when no treatment is operated on the drug containers, so as to maintain said enclosed chamber and enclosed compartment thermally sealed.
